# EUROPÄISCHE PATENTSCHRIFT

(11) **EP 1 158 042 B1**
(45) Veröffentlichungstag und Bekanntmachung des Hinweises auf die Patenterteilung: **02.01.2008**
(21) Anmeldenummer: 01112454.2
(22) Anmeldetag: 22.05.2001
(51) Int. Cl.: C12M 1/12, C12M 1/40

(54) **Verfahren zur biotechnologischen Erzeugung von Duft- und/oder Aromastoffen**
Process for the biotechnological preparation of flavoring or fragrance products
Procédé pour la préparation biotechnologique de composés aromatiques ou parfumants

(30) Priorität: 22.05.2000 DE 10024962
(43) Veröffentlichungstag der Anmeldung: 28.11.2001
(73) Patentinhaber: DECHEMA Gesellschaft für Chemische Technik und Biotechnologie e.V., 60486 Frankfurt (DE)
(72) Erfinder: Blümke, Wilfried, Dr., 61137 Schöneck (DE); Schrader, Jens, Dr., 60316 - Frankfurt am Main (DE)
(74) Vertreter: Meyer-Dulheuer, Karl-Hermann

(56) Entgegenhaltungen:
- EP-A- 0 164 608
- GB-A- 2 324 257
- JP-A- 5 137 969
- US-A- 4 326 036
- PATENT ABSTRACTS OF JAPAN vol. 010, no. 185 (C-357), 27. Juni 1986 (1986-06-27) & JP 61 035805 A (NITTO ELECTRIC IND CO LTD), 20. Februar 1986 (1986-02-20)

## Beschreibung

Die Erfindung betrifft ein Verfahren zur biotechnologischen Erzeugung von Duft- und/oder Aromastoffen, umfassend eine Bioreaktoreinrichtung, in der in einer Flüssigphase infolge mikroorganismen- und/oder enzyminduzierter Stoffumwandlungsprozesse die Duft- und/oder Aromastoffe erzeugt werden, sowie eine angeschlossene Pervaporationseinrichtung, in der die Zielprodukte kontinuierlich oder sequentiell aus der Flüssigphase isoliert werden, wobei das Retentat, das die duft-und/oder aromastoffabgereicherte Phase darstellt, wenigstens teilweise in die Bioreaktoreinrichtung zurückgeführt wird.

Bei der biotechnologischen Erzeugung von Duft- und/oder Aromastoffen sind die ungenügende Konzentration der erzeugten Stoffe, die Produktivität und die Ausbeute des dafür herangezogenen biochemischen Produktionsprozesses ein wesentliches Problem. Ursache hierfür ist regelmäßig die starke Inhibition von Fermentationsprodukten, bspw. der Duft- und Aromastoffe, auf die sie erzeugenden Mikroorganismen.

Bisher wurden Duft- und Aromastoffe mit satzweise betriebenen Bioreaktoreinrichtungen mit anschließender Zellseparation und Aufarbeitung der Flüssigphase zur Stoffisolierung verwendet. Aus der WO-A-96 22 381 ist ein standardmäßiges Trennverfahren bekannt, das auf destillativer bzw. extraktiver Basis arbeitet, um vertriebsfähige Reinheitsstufen von Aromastoffen zu erzielen.

Auch die sorptive Trennung verschiedener Aromastoffe auf Basis von Ionenaustauschern und anderen organischen und anorganischen Materialien, wie z.B. Silikonharzen und Zeolithen, sind bekannt, bspw. aus der EP-A-0 979 806. Neben den vorbeschriebenen klassischen Verfahren können extraktive und sorptive Verfahren auch direkt zur kontinuierlichen Stoffabtrennung eingesetzt werden.

Die bekannten Verfahren weisen erhebliche Nachteile auf.
So ist z.B. im Anschluß an eine Fermentation eine Destillation erforderlich, die einen hohen Aufwand an Energie erfordert, mit der auch für die erzeugten Stoffe zusätzlich nachteiligen Folge, daß nämlich die zu erzeugenden Stoffe thermisch stark belastet werden, was (beschleunigte Durchbruchskinetik). Auch die Desorption führt häufig zu einer thermischen Belastung der Stoffe, zu einer Verunreinigung des Sorptionsmittel durch LM sowie zu einer begrenzten Lebensdauer des Extraktionsmittels, bspw. durch Auftreten von Fouling durch Belegung der Oberfläche.

Alle vorangehend beschriebenen Verfahren weisen zudem den Nachteil auf, daß eine vorherige Abtrennung von Biokatalysatoren von der Flüssigphase notwendig ist, wenn eine kontinuierliche Stoffisolierung angestrebt wird. Bekannte Filtrationstechniken für die Zellrückhaltung führen zu erhöhten Investitionskosten sowie zusätzlichen technischen Problemen aufgrund der hohen Anfälligkeit gegenüber Fouling/Verblockung. Schließlich treten auch insgesamt Probleme in bezug auf eine sterile Verfahrensführung auf.

In US-A-4 326 036 wird ein Verfahren zur Herstellung von Ethanol aus Zuckerrohr beschrieben, wobei nach erfolgter mechanischer Zerkleinerung des Zuckerrohrs fermentierbare Zucker einer Ethanol-Fermentationsanlage zugeführt werden, in der unter Einwirkung von Mikroorganismen Ethanol gebildet und durch eine Einheit über Membrantrennung abgeführt wird. Gemäß der JP 05 137969 wird ein Verfahren zur Abtrennung von Ethanol aus einer 10%-igen wässrigen Ethanollösung über eine für Alkohol permeable Membran dargestellt.

Ein Verfahren zur Abtrennung von Produkten und/oder Wirkstoffen aus einem Produkt-Substrat-Gemisch mit Hilfe einer Pervaporationsmembran wird gemäß der DE 34 184 14 beschrieben.

In DE-C-44 21 682 wird der Einsatz der Pervaporation zur Abtrennung von Aromen aus wäßrigen Flüssigkeiten beschrieben, womit ein Teil der zuvor beschriebenen Nachteile überwunden wird. Allerdings ist der dort beschriebene Einsatz der Membranen unvorteilhaft wegen der mangelnden Effektivität des Membranverfahrens beim Einsatz am aktiven Bioreaktor, weil große Membranflächen benötigt werden, um die produzierten Duft- und/oder Aromastoffe abzutrennen. Dies führt zu unverhältnismäßig hohem Aufwand und Kosten.

Es ist somit Aufgabe der vorliegenden Erfindung, ein Verfahren der eingangs genannten Art zu schaffen, mit dem Duft- und Aromastoffe mit hoher Effektivität, d.h. hoher Produktivität und Ausbeute, erzeugt werden können und inhibierende Einflüsse auf die verfahrensmäßig erzeugten Duft- und Aromastoffe während des Verfahrensverlaufs vermieden werden und die Nachteile im Stand der Technik bekannter Verfahren vermieden werden sollen, wobei das Verfahren einfach, effektiv und kostengünstig durchführbar sein soll.

Gelöst wird die Aufgabe gemäß der Erfindung dadurch, daß das Verfahren bei Prozeßtemperaturen > 25° C unter Verwendung thermotoleranter und/oder thermophiler Mikroorganismen und/oder Enzymen durchgeführt wird, wobei der für die Pervaporation in der Pervaporationseinrichtung erforderliche Unterdruck durch eine in der Permeatleitung für das Permeat in Reihe geschaltete Vakuumpumpe erzeugt wird. Das heißt mit anderen Worten, daß die Flüssigphase bei einer erhöhten Prozeßtemperatur > 25° C auf eine Pervaporationseinrichtung gegeben wird, in der die Flüssigphase in ein Permeat und ein Retentat getrennt wird, wobei das Permeat die duft- und/oder aromastoffangereicherte Phase darstellt und wobei das Retentat die duft- und/oder aromastoffabgereicherte Phase darstellt, die in die Bioreaktoreinrichtung wenigstens teilweise zurückgeführt wird.

Die im Vergleich zu konventionellen Bioprozessen erhöhte Prozeßtemperatur - ermöglicht durch den Einsatz geeigneter thermotoleranter und/oder thermophiler Mikroorganismen und/oder Enzymen - ist der Grund dafür, daß der Gesamtprozeß eine deutliche Effizienzsteigerung erfährt und somit die Wirtschaftlichkeit eines solchen Verfahrens signifikant verbessert wird. Die erhöhte Prozeßtemperatur führt zu größeren Stoffflüssen durch die Membran, wodurch erst der Einsatz solcher Mikroorganismen ermöglicht wird, die auf maximale Produktivität optimierte Leistungsstämme darstellen, da diese ansonsten durch die Akkumulation der gewünschten Zielprodukte in der Kulturflüssigkeit in relativ kurzer Zeit inhibiert würden. Ebenso ist bei Verwendung von Enzympräparaten eine positive Beeinflussung der Raktionskinetik durch die verbesserte Abfuhr des Produktes zu erwarten. Durch Wahl der Betriebsbedingungen der Pervaporation, bspw. durch Variation des permeatseitigen Druckes, der stofflichen Zusammensetzung der Membran, der Temperatur der zugeführten Flüssigphase, der Strömung der Flüssigphase, den eigentlichen Werkstoffen, aus denen die Pervaporationseinrichtung gefertigt wird, der Membranstruktur, der Membranfläche und der Membrandicke, wird über Konzentration und Zusammensetzung der abgeführten Produkte und damit über die stoffliche Zusammensetzung des ganz oder teilweise in den Bioreaktor zurückgeführten Retentatstroms auf die biochemischen Stoffumwandlungsprozesse im Bioreaktor Einfluß genommen und somit die Membrantrenneinrichtung zur Steuerung des integrierten Bioprozesses eingesetzt. Dies kann beispielsweise dazu führen, daß Wertstoffe gewonnen werden, die in konventionellen Bioprozessen mit nachgeschalteter Produktaufreinigung nicht isolierbar sind.

Ein weiterer Vorteil des erfindungsgemäßen Verfahrens liegt im wesentlichen darin, daß durch den Einsatz der Membrantrenneinrichtung quasi ein geschlossenes System zusammen mit der Bioreaktoreinrichtung geschaffen wird, d.h. die Membrantrenneinrichtung kann mit der in der Bioreaktoreinrichtung erzeugten Flüssigphase ungefiltert beaufschlagt werden, wobei eine sterile Rückführung des Retentats in die Bioreaktoreinrichtung erfolgt. Es werden somit typische Inhibitionseffekte durch Stoffakkumulation vermieden, wobei der Stoff im Permeat als hochangereichertes Konzentrat bereitgestellt werden kann. Die Permeatmenge ist im Vergleich zum Volumen der Flüssigphase sehr klein, bspw. < 10 %, wodurch sich eine sehr leichte Aufarbeitung des Permeats zur Reinsubstanz ergibt. Das Permeat ist als Matrix für die Aufarbeitung deutlich weniger komplex als die von dem Bioreaktor gelieferte Flüssigphase, da im Permeat nur Substanzen mit signifikantem Partialdruck vorliegen, d.h. im wesentlichen Wasser und die gewünschten Stoffe sowie ggf. verdampfbare organische Nebenprodukte, d.h. keine Feststoffe, Proteine, Salze usw..

Die Membran in der Membrantrenneinrichtung fungiert gleichzeitig als Trennmedium und Sterilbarriere, so daß keine vorherige Zell- und/oder Enzymabtrennung nötig ist.

Die Stoffe, die durch die Membrantrenneinrichtung nicht abgetrennt werden, verbleiben in der Bioreaktoreinrichtung bzw. werden in diese zurückgeführt. Erfindungsgemäß werden zudem vorteilhafterweise inhibierende Stoffe entfernt, sofern sie sich mit der Membrantrenneinrichtung abtrennen lassen, und zwar zusätzlich zu den gewünschten Duft- und Aromastoffen, wodurch ebenfalls eine Verbesserung der Effizienz des Herstellungsverfahrens möglich ist.

Der Einsatz thermotoleranter und/oder thermophiler Mikroorganismen und/oder Enzymen bei Prozeßtemperaturen von vorzugsweise ≥ 35° C ermöglicht eine weitere Effizienzsteigerung der Leistung des gesamten Prozesses. Durch Wahl geeigneter Betriebsbedingungen der Pervaporationseinrichtung wird über die stoffliche Zusammensetzung des ganz oder teilweise in den Bioreaktor zurückgeführten Retentatstroms auf die biochemischen Stoffumwandlungsprozesse im Bioreaktor Einfluß genommen und somit die Pervaporationseinrichtung zur Steuerung der Bioreaktoreinrichtung eingesetzt.

Durch die erfindungsgemäße Lösung kann eine effektive Abtrennung der produzierten Duft- und/oder Aromastoffe durch die Membrantrenneinrichtung erreicht werden, weil höhere Temperaturen, beispielsweise > 25° C, vorzugsweise ≥ 35° C, für auch aus ökonomischer Sicht realisierbare Bedingungen sorgen, mit denen die Inhibition durch die produzierten Duft- und Aromastoffe entscheidend verringert werden kann.

Um die Eigenschaften der Pervaporationseinrichtung zu verbessern, kann es vorteilhaft sein, die von der Bioreaktoreinrichtung in die Membrantrenneinrichtung geführte Flüssigphase vor Eintritt in die Membraneinrichtung zu temperieren. Dazu kann bspw. ein Wärmetauscher herangezogen werden, der die Flüssigkeitsphase vor Eintritt in die Membrantrenneinrichtung erwärmt bzw. eine Temperierung in Abhängigkeit der geeignetsten Trenntemperatur in der Membrantrenneinrichtung vornimmt.

Gleichermaßen kann es vorteilhaft sein, die die Membrantrenneinrichtung verlassende, von Duft- und Aromastoffen abgereicherte Flüssigphase, d.h. das Retentat, vor Zuführung in die Bioreaktoreinrichtung zu temperieren, um auch die Flüssigphase in der Bioreaktoreinrichtung auf einem vorbestimmbaren Temperaturniveau zu halten. Das kann durch Erwärmung oder Wärmeentzug des Retentats erfolgen.

Alternativ kann die von der Bioreaktoreinrichtung in die Membrantrenneinrichtung geführte Flüssigphase vor Eintritt in die Membrantrenneinrichtung über eine Zell-und/oder Enzymrückhaltevorrichtung geleitet werden, wodurch eine Rückhaltung der aus der Bioreaktoreinrichtung zugeführten Zellen und/oder Enzyme erfolgt mit der Wirkung, daß dadurch ein Aktivitätsverlust und eine Schädigung der Mikroorganismen und/oder Enzyme bei der Passage durch die Membrantrenneinrichtung vermieden werden.

Die Zell- und/oder Enzymrückhaltevorrichtung kann gemäß einer anderen vorteilhaften Ausgestaltung des Verfahrens wiederum eine Membrantrenneinrichtung sein, beispielsweise eine Filtrationseinheit, deren erzeugte zell-und/oder enzymfreie Flüssigphase in die Membrantrenneinrichtung (Pervaporationseinrichtung) geleitet wird und deren zell- und/oder enzymhaltige Flüssigphase in die Bioreaktoreinrichtung geleitet wird.

Grundsätzlich werden der Trennprozeß der Flüssigphase in der Membrantrenneinrichtung und der biochemische Stoffumwandlungsprozeß in der Bioreaktoreinrichtung vorzugsweise im Bereich gleicher Temperaturen betrieben, es ist aber auch vorzugsweise möglich, die Temperatur der Flüssigphase in der Membrantrenneinrichtung von der Temperatur des biochemischen Stoffumwandlungsprozesses in der Bioreaktoreinrichtung zu trennen, d.h. eine Einstellung der Temperatur für den biochemischen Stoffumwandlungsprozeß unabhängig von der Temperatur des Membrantrennprozesses zu wählen, um für beide Verfahrensbereiche die geeignetste Temperatur auszuwählen, um die Effektivität des Gesamtverfahrens auf das technisch Machbare zu optimieren, mit dem Ziel, die Kultivierung der Mikroorganismen im biochemischen Stoffumwandlungsprozeß bei der dafür optimalen Temperatur durchzuführen und für die Trennung der Flüssigphase in der Membrantrenneinrichtung die Temperatur zu wählen, mit der das Membrantrennverfahren am effektivsten durchgeführt werden kann.

Sehr vorteilhaft ist es für die Verfahrensführung ebenfalls, die im biochemischen Stoffumwandlungsprozeß in der Bioreaktoreinrichtung erzeugte und/oder der Bioreaktoreinrichtung zum biochemischen Stoffumwandlungsprozeß zugeführte Wärme dem Trennprozeß in der Membrantrenneinrichtung (Pervaporationseinrichtung) zuzuführen, falls für die Optimierung des Verfahrens dafür ein Bedürfnis besteht. Es wird somit zusätzliche Energiezufuhr von außen entbehrlich, was wiederum zu einer Effektivitätssteigerung der Durchführung des Verfahrens führen kann.

Auch das in die Bioreaktoreinrichtung zurückgeführte Retentat wird vorzugsweise zur Steuerung des biochemischen Stoffumwandlungsprozesses in der Bioreaktoreinrichtung verwendet, wobei durch die Wahl der Betriebsbedingungen des Membrantrennprozesses die stoffliche Zusammensetzung des Retentats einstellbar ist.

Grundsätzlich kann die Stofftrennung in der Membrantrenneinrichtung bei laufendem Fermentationsprozeß kontinuierlich, vorzugsweise aber auch diskontinuierlich betrieben werden, d.h. das Verfahren kann an die Menge der erzeugten Duft- und Aromastoffe angepaßt betrieben werden, d.h. je nach Anfall der Menge der erzeugten Duft- und Aromastoffe im Fermentationsprozeß.

Die Erfindung wird nun unter Bezugnahme auf die nach-folgenden schematischen Zeichnungen anhand mehrerer unterschiedlicher Vorrichtungen zur Ausführung des Verfahrens eingehend beschrieben. Darin zeigen:
- Fig. 1: in Form eines Blockschaltbildes eine erste Ausgestaltung einer Vorrichtung zur Ausführung des Verfahrens,
- Fig. 2: eine zweite Ausgestaltung der Vorrichtung zur Ausführung des Verfahrens, bei der in der Zuführung der Flüssigphase (Feed) zur Membrantrenneinrichtung und in der Rückführung des Retentats zur Bioreaktoreinrichtung jeweils Wärmetauscher angeordnet sind, und
- Fig. 3: eine Vorrichtung zur Ausführung des Verfahrens gemäß Fig. 2, bei der in der Zuführung der Flüssigphase (Feed) zur Membrantrenneinrichtung eine Vorrichtung zur Rückhaltung von Zellen und/oder Enzymen angeordnet ist.

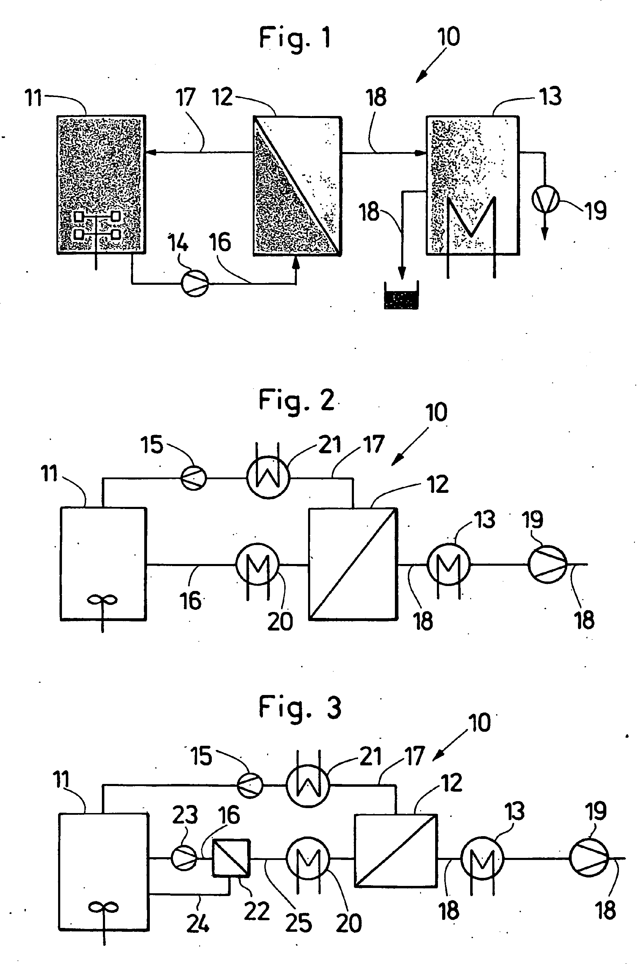

Es wird zunächst Bezug genommen auf die Darstellung gemäß Fig. 1, in der eine Vorrichtung 10 zur Ausführung des Verfahrens gemäß der Erfindung in ihrer Grundkonzeption dargestellt ist. Eine Bioreaktoreinrichtung 11, in der ein biochemischer Stoffumwandlungsprozeß stattfindet, bei dem Duft- und/oder Aromastoffe erzeugt werden, ist über eine Ausgangsleitung mit dem Eingang einer Membrantrenneinrichtung 12 (Pervaporationseinrichtung) verbunden, in der Flüssigphase 16 (Feed), ggf. über eine zwischengeschaltete Pumpeneinrichtung 14, der Membrantrenneinrichtung 12 zugeführt wird. Die Membrantrenneinrichtung 12 weist einen Ausgang für das Permeat 18 und einen Ausgang für das Retentat 17 auf. Das Retentat 17 wird wenigstens teilweise zurückgeführt.

Alternativ zur gezeigten Position der Pumpeinrichtung 14 kann diese auch in die Retentatleitung 17 zwischengeschaltet sein (Pumpeneinrichtung 15).

Neben der baulichen Trennung von Bioreaktoreinrichtung 11 und Membrantrenneinrichtung 12 kann auch ein komplett integrierter Ansatz in Form einer Art Membranreaktor zur Produktion der Duft- und/oder Aromastoffe verwendet werden, wobei sich die Pervaporationseinrichtung in oder direkt an der Bioreaktoreinrichtung befindet.

Die Membrantrenneinrichtung 12 ist mit einem Wärmetauscher 13 verbunden, wobei das Permeat 18, im Wärmetauscher 13 auskondensiert wird. Der für die Pervaporation in der Membrantrenneinrichtung 12 erforderliche Unterdruck wird durch eine in der Permeatleitung für das Permeat 18 in Reihe geschaltete Vakuumpumpe 19 erzeugt.

Um den biochemischen Herstellungsprozeß in der Bioreaktoreinrichtung 11 geeignet steuern zu können, kann durch Variation der Betriebsbedingungen der Membrantrenneinrichtung (Pervaporationseinrichtung), z.B. über die Vorgabe des permeatseitigen Druckes mittels der Vakuumpumpe, die stoffliche Zusammensetzung des Permeats 18 und damit das ganz oder teilweise in die Bioreaktoreinrichtung 11 zurückgeführten Retentats 17 eingestellt werden.

Die Vorrichtung 10 gemäß Fig. 2 unterscheidet sich von der gemäß Fig. 1 dadurch, daß in den Leitungsverbindungen zwischen der Bioreaktoreinrichtung 11 und der Membrantrenneinrichtung 12 sowohl für das Feed 16 als auch für das Retentat 17 jeweils ein Wärmetauscher 20, 21 angeordnet sind. Diese dienen zur Einstellung der geeigneten Temperatur für die Flüssigphase 16 und das Retentat 17. Die Verwendung beider Wärmetauscher 20, 21 ist nicht zwingend notwendig, vielmehr kann auch jeder Wärmetauscher 20, 21 für sich allein in der Vorrichtung 10 angeordnet sein.

Die Vorrichtung 10 gemäß Fig. 3 unterscheidet sich von der gemäß Fig. 2 dadurch, daß in der Leitung, über die von der Bioreaktoreinrichtung 11 zur Membrantrenneinrichtung 12 die Flüssigphase 25 der Membrantrenneinrichtung 12 zugeführt wird, eine Zell- und/oder Enzymrückhaltevorrichtung 22 angeordnet ist. Die Zell-und/oder Enzymrückhaltevorrichtung 22 ist derart geschaltet, daß die darin erzeugte enzymhaltige Flüssigkeit in die Membrantrenneinrichtung 12 zugeführt wird,
wobei die zell- und/oder enzymhaltige Flüssigphase 24 in die Bioreaktoreinrichtung 11 zurückgeführt wird. Die für den Betrieb der Zell- und/oder Enzymrückhaltevorrichtung notwendige Kreislaufführung wird durch eine Pumpeneinrichtung 23 gewährleistet.

Das Verfahren zur biotechnologischen Erzeugung von Duft-und/oder Aromastoffen läuft folgendermaßen ab: In der Bioreaktoreinrichtung 11 werden in einer Flüssigphase infolge biochemischer Stoffumwandlungsprozesse die Duft-und/oder Aromastoffe erzeugt. Dazu wird die Bioreaktoreinrichtung 11 mit geeigneten Mikroorganismen und/oder Enzymen, die thermotolerant und/ oder thermophil sind, beladen und mit einer geeigneten Nährlösung versetzt.

Infolge der biochemischen Stoffumwandlungsprozesse wird eine Flüssigphase gebildet, in der die Duft- und/oder Aromastoffe enthalten sind. Diese Flüssigphase 16 (Feed) wird auf die Membrantrenneinrichtung 12 gegeben, in der die Flüssigphase 16 in ein Permeat 18 und in ein Retentat 17 auf an sich bekannte Weise mittels Pervaporation getrennt wird. Das Permeat 18, das die duft- und/oder aromastoffangereicherte Phase darstellt, wird membranrückseitig abgeführt, wohingegen das Retentat 17, das die duft- und/oder aromastoffabgereicherte Phase darstellt, in die Bioreaktoreinrichtung 11 zurückgegeben wird.

Das Permeat 18 wird im Wärmetauscher 13, der einen Kondensator darstellt, auskondensiert.

Die Betriebsbedingungen der ersten Membrantrenneinrichtung 12 können durch Variation der Temperatur der zugeführten Flüssigphase 16, der Strömung der Flüssigphase 16, dem Druck des Permeats 18, der eigentlichen Werkstoffe, die die Membrantrenneinrichtung 12 (Pervaporationseinrichtung) binden, der Membranstruktur, der Membranfläche und der Membrandicke eingestellt werden, was gleichermaßen für die Betriebsparameter des Wärmetauschers 13 (Kondensators) gilt.

Das Membrantrennverfahren kann kontinuierlich und diskontinuierlich in Abhängigkeit des laufenden biochemischen Stoffumwandlungsprozesses betrieben werden.

### Beispiel 1:

Fed-Kultivierung (Glucose) von S. cerevisiae in synthetischem Medium bei pH 5,9 mit 2g/L Phe für die Produktion von 2-Phenylethanol:
Ohne Membrantrenneinrichtung:
volumetrische Produktivität 17,0 g/(L·h)
Mit Membrantrenneinrichtung:
volumetrische Produktivität 23,4 g/(L·h)

### Beispiel 2:

Biokonversion von L-Phenylalanin zu 2-Phenylethanol und 2-Phenylethylacetat mit einer thermotoleranten Hefe der Gattung Kluyveromyces bei einer Prozeßtemperatur von 40°C. Die Aminosäure wurde einem semisynthetischen Grundmedium als Precursor vor Beginn der Fermentation im Überschuß zugeführt. Nach 24 h wurden insgesamt 1,3 g/L 2-Phenylethanol und 1,1 g/L 2-Phenylethylacetat im Permeat gewonnen. Als Membran wurde eine Kompositmembran mit Poly-Okty-methyl-Siloxan als trennaktiver Schicht auf PolyEtherImid verwendet. Neben der erhöhten Produktivität durch die Verwendung eines thermotoleranten Hefestammes zeigt dieses Beispiel die Verfahrenssteuerungsmöglichkeiten über die Einstellung der Pervaporationsbedingungen.

Es hat sich gezeigt, daß nur mit dem erfindungsgemäßen Verfahren für den gewählten Organismus die Substanz 2-Phenylethylacetat in signifikanten Ausbeuten zu gewinnen ist.

### Bezugszeichenliste

- 10: Vorrichtung
- 11: Bioreaktoreinrichtung
- 12: Membrantrenneinrichtung
- 13: Wärmetauscher
- 14: Pumpeneinrichtung
- 15: Pumpeneinrichtung
- 16: Flüssigphase (Feed)
- 17: Retentat
- 18: Permeat
- 19: Vakuumpumpe
- 20: Wärmetauscher
- 21: Wärmetauscher
- 22: Zell- und/oder Enzymrückhaltevorrichtung
- 23: Pumpeneinrichtung
- 24: zell- und/oder enzymhaltige Flüssigphase (Feed)
- 25: zell- und/oder enzymfreie Flüssigphase (Feed)

## Patentansprüche

1. Verfahren zur biotechnologischen Erzeugung von Duft-und/oder Aromastoffen, umfassend eine Bioreaktoreinrichtung, in der in einer Flüssigphase infolge mikroorganismen- und/oder enzyminduzierter Stoffumwandlungsprozesse die Duft- und/oder Aromastoffe erzeugt werden, sowie eine angeschlossene Pervaporationseinrichtung, in der die Zielprodukte kontinuierlich oder sequentiell aus der Flüssigphase isoliert werden, wobei das Retentat, das die duft- und/oder aromastoffabgereicherte Phase darstellt, wenigstens teilweise in die Bioreaktoreinrichtung zurückgeführt wird, **dadurch gekennzeichnet, daß** das Verfahren bei Prozeßtemperaturen > 25° C unter Verwendung thermotoleranter und/oder thermophiler Mikroorganismen und/oder Enzymen durchgeführt und der für die Pervaporation in der Pervaporationseinrichtung erforderliche Unterdruck durch eine in der Permeatleitung für das Permeat in Reihe geschaltete Vakuumpumpe erzeugt wird.

2. Verfahren nach Anspruch 1, **dadurch gekennzeichnet, daß** die Prozeßtemperatur 35° C ist.

3. Verfahren nach einem oder beiden der Ansprüche 1 oder 2, **dadurch gekennzeichnet, daß** durch Wahl der Betriebsbedingungen der Pervaporation, vorzugsweise durch Variation des permeatseitigen Druckes und/oder der stofflichen Zusammensetzung der Membran, über die Konzentration und Zusammensetzung der abgeführten Produkte und damit der stofflichen Zusammensetzung des ganz oder teilweise in den Bioreaktor zurückgeführten Retentatstroms auf die biochemischen Stoffumwandlungsprozesse der Mikroorganismen und/oder Enzyme im Bioreaktor Einfluß genommen und somit die Pervaporationseinrichtung zur Steuerung des integrierten Bioprozesses eingesetzt wird.

4. Verfahren nach einem oder mehreren der Ansprüche 1 bis 3, **dadurch gekennzeichnet, daß** die von der Bioreaktoreinrichtung in die Pervaporationseinrichtung geführte Flüssigphase vor Eintritt in die Pervaporationseinrichtung temperiert wird.

5. Verfahren nach einem oder mehreren der Ansprüche 1 bis 4, **dadurch gekennzeichnet, daß** die die Pervaporationseinrichtung verlassende, von Duft-und/oder Aromastoffen abgereicherte Flüssigphase vor Zurückführung in die Bioreaktoreinrichtung temperiert wird.

6. Verfahren nach einem oder mehreren der Ansprüche 1 bis 5, **dadurch gekennzeichnet, daß** die von der Bioreaktoreinrichtung in die Pervaporationseinrichtung geführte Flüssigphase vor Eintritt in die Pervaporationseinrichtung über eine Zell-und/oder Enzymrückhaltevorrichtung geleitet wird.

7. Verfahren nach einem oder mehreren der Ansprüche 1 bis 6, **dadurch gekennzeichnet, daß** die zell- und/oder enzymfreie Flüssigkeit aus der Zell- und/oder Enzymrückhaltevorrichtung in die Pervaporationseinrichtung geleitet wird und die zell-und/oder enzymhaltige Flüssigkeit aus der Zell- und/oder Enzymrückhaltevorrichtung wenigstens teilweise in die Bioreaktoreinrichtung geleitet wird.

8. Verfahren nach einem oder mehreren der Ansprüche 1 bis 7, **dadurch gekennzeichnet, daß** der Trennprozeß der Flüssigphase in der Pervaporationseinrichtung und der biochemische Stoffumwandlungsprozeß in der Bioreaktoreinrichtung im Bereich gleicher Temperaturen betrieben werden.

9. Verfahren nach einem oder mehreren der Ansprüche 1 bis 7, **dadurch gekennzeichnet, daß** der Trennprozeß der Flüssigphase in der Membrantrenneinrichtung und der biochemische Stoffumwandlungsprozeß in der Bioreaktoreinrichtung im Bereich verschiedener Temperaturen betrieben werden.

10. Verfahren nach einem oder mehreren der Ansprüche 1 bis 9, **dadurch gekennzeichnet, daß** die im biochemischen Stoffumwandlungsprozeß in der Bioreaktoreinrichtung erzeugte und/oder der Bioreaktoreinrichtung zum biochemischen Stoffumwandlungsprozeß zugeführten Wärme dem Trennprozeß in der Pervaporationseinrichtung zugeführt wird.

11. Verfahren nach einem oder mehreren der Ansprüche 1 bis 10, **dadurch gekennzeichnet, daß** die Stofftrennung in der Membrantrenneinrichtung bei laufendem biochemischen Stoffumwandlungsprozeß kontinuierlich betrieben wird.

12. Verfahren nach einem oder mehreren der Ansprüche 1 bis 10, **dadurch gekennzeichnet, daß** die Stofftrennung in der Membrantrenneinrichtung bei laufendem biochemischen Stoffumwandlungsprozeß diskontinuierlich betrieben wird.

## Claims

1. A process for the biotechnological production of odorous and/or aromatic substances, comprising a bioreactor device in which the odorous and/or aromatic substances are produced in a liquid phase as a result of micro-organism- and/or enzyme-induced material conversion processes, and a pervaporation device in which the target products are continuously or sequentially isolated from the liquid phase, wherein the retentate comprising the phase depleted in odorous and/or aromatic substances, is at least partially returned to the bioreactor device, **characterised in that** the process is carried out at process temperatures > 25°C using thermotolerant and/or thermophilic micro-organisms and/or enzymes and the negative pressure required for the pervaporation in the pervaporation device is generated by a vacuum pump connected in series in the permeate line for the permeate.

2. The process according to claim 1, **characterised in that** the process temperature is 35°C.

3. The process according to one or both of claims 1 or 2, **characterised in that** by selecting the operating conditions of the pervaporation, preferably by varying the permeate-side pressure and/or the substance composition of the membrane, the biochemical material conversion processes of the micro-organisms and/or enzymes in the bioreactor can be influenced via the concentration and composition of the removed products and therefore the substance composition of the retentate stream returned completely or partially to the bioreactor, and consequently the pervaporation device is used to control the integrated bioprocess.

4. The process according to one or more of claims 1 to 3, **characterised in that** the liquid phase fed from the bioreactor device into the pervaporation device is temperature controlled before entry into the pervaporation device.

5. The process according to one or more of claims 1 to 4, **characterised in that** the liquid phase leaving the pervaporation device, which is depleted in odorous and/or aromatic substances, is temperature-controlled before returning to the bioreactor device.

6. The process according to one or more of claims 1 to 5, **characterised in that** the liquid phase fed from the bioreactor device to the pervaporation device is passed over a cell and/or enzyme retention device before entry into the pervaporation device.

7. The process according to one or more of claims 1 to 6, **characterised in that** the cell- and/or enzyme-free liquid is fed from the cell and/or enzyme retention device into the pervaporation device and the cell-and/or enzyme-containing liquid is fed from the cell and/or enzyme retention device at least partly into the bioreactor device.

8. The process according to one or more of claims 1 to 7, **characterised in that** the separation process of the liquid phase in the pervaporation device and the biochemical material conversion process in the bioreactor device are operated in the same temperature range.

9. The process according to one or more of claims 1 to 7, **characterised in that** the separation process of the liquid phase in the pervaporation device and the biochemical material conversion process in the bioreactor device are operated in different temperature ranges.

10. The process according to one or more of claims 1 to 9, **characterised in that** the heat generated in the biochemical material conversion process in the bioreactor device and/or supplied to the bioreactor device for the biochemical material conversion process is fed to the separating process in the pervaporation device.

11. The process according to one or more of claims 1 to 10, **characterised in that** the material separation in the membrane separating device is operated continuously during the ongoing biochemical material conversion process.

12. The process according to one or more of claims 1 to 10, **characterised in that** the material separation in the membrane separating device is operated discontinuously during the ongoing biochemical material conversion process.

## Revendications

1. Procédé de production biotechnologique de parfums et/ou d'arômes comprenant un dispositif de type bioréacteur dans lequel lesdits parfums et/ou arômes sont produits en phase liquide suite à des processus métaboliques induits par des micro-organismes et/ou des enzymes, ledit bioréacteur étant relié à un dispositif de pervaporation dans lequel les produits recherchés sont isolés de la phase liquide de manière continue ou séquentielle, le rétentat constitué par la phase appauvrie en parfums/arômes étant au moins partiellement recyclé dans le dispositif de type bioréacteur, **caractérisé en ce que** le procédé se déroule à des températures de processus > 25 °C en utilisant des micro-organismes et/ou enzymes thermotolérants et/ou thermophiles et que la pression négative requise dans le dispositif de pervaporation pour effectuer la pervaporation est générée par une pompe à vide montée en série dans le conduit de perméat destiné au perméat.

2. Procédé selon la revendication 1, **caractérisé en ce que** la température de processus est de 35 °C.

3. Procédé selon l'une des deux revendications 1 ou 2, **caractérisé en ce que** les processus métaboliques biochimiques des micro-organismes et/ou enzymes dans le bioréacteur sont influencés à travers le choix des conditions opératoires de la pervaporation, de préférence en variant de la pression du côté perméat et/ou de la composition matérielle de la membrane, à travers la concentration et la composition des produits prélevés et, par conséquent, la composition matérielle du flux de rétentat qui est partiellement ou en totalité recyclé dans le bioréacteur, le dispositif de pervaporation étant ainsi utilisé pour commander le bioprocessus intégré.

4. Procédé selon l'une ou plusieurs des revendications 1 à 3, **caractérisé en ce que** la phase liquide en provenance du dispositif de type bioréacteur est mise en température avant d'être introduite dans le dispositif de pervaporation.

5. Procédé selon l'une ou plusieurs des revendications 1 à 4, **caractérisé en ce que** la phase liquide appauvrie en parfums et/ou d'arômes est mise en température à la sortie du dispositif de pervaporation avant d'être recyclée dans le dispositif de type bioréacteur.

6. Procédé selon l'une ou plusieurs des revendications 1 à 5, **caractérisé en ce que** la phase liquide en provenance du dispositif de type bioréacteur passe à travers un dispositif de rétention de cellules et/ou d'enzymes avant d'être introduite dans le dispositif de pervaporation.

7. Procédé selon l'une ou plusieurs des revendications 1 à 6, **caractérisé en ce que** le liquide exempt de cellules et/ou d'enzymes en provenance du dispositif de rétention de cellules et/ou d'enzymes est introduit dans le dispositif de pervaporation et que le liquide contenant des cellules et/ou des enzymes en provenance du dispositif de rétention de cellules et/ou d'enzymes est au moins partiellement introduit dans le dispositif de type bioréacteur.

8. Procédé selon l'une ou plusieurs des revendications 1 à 7, **caractérisé en ce que** le processus de séparation de la phase liquide se déroulant dans le dispositif de pervaporation et le processus métabolique biochimique se déroulant dans le dispositif de type bioréacteur sont réalisés dans la même gamme de températures.

9. Procédé selon l'une ou plusieurs des revendications 1 à 7, **caractérisé en ce que** le processus de séparation de la phase liquide se déroulant dans le dispositif de séparation membranaire et le processus métabolique biochimique se déroulant dans le dispositif de type bioréacteur sont réalisés dans différentes gammes de températures.

10. Procédé selon l'une ou plusieurs des revendications 1 à 9, **caractérisé en ce que** la chaleur générée dans le processus métabolique biochimique se déroulant dans le dispositif de type bioréacteur ou apportée au dispositif de type bioréacteur pour effectuer le processus métabolique biochimique est transférée au processus de séparation se déroulant dans le dispositif de pervaporation.

11. Procédé selon l'une ou plusieurs des revendications 1 à 10, **caractérisé en ce que** la séparation des matières se déroulant dans le dispositif de séparation membranaire est réalisée parallèlement au processus métabolique biochimique de manière continue.

12. Procédé selon l'une ou plusieurs des revendications 1 à 10, **caractérisé en ce que** la séparation des matières se déroulant dans le dispositif de séparation membranaire est réalisée parallèlement au processus métabolique biochimique de manière discontinue.
